# EUROPEAN PATENT APPLICATION

(11) **EP 4 643 937 A1**
(43) Date of publication of application: **05.11.2025**
(21) Application number: 24172916.9
(22) Date of filing: 29.04.2024
(51) Int. Cl.: A61N 1/375

(54) **BONDING OF COMPONENTS AND ASSEMBLIES IN PERMANENT IMPLANTS WITH THERMALLY ACTIVATABLE POLYURETHANE FILMS**

(71) Applicant: BIOTRONIK SE & Co. KG, 12359 Berlin (DE)
(72) Inventor: Egart, Boris, 13353 Berlin (DE)
(74) Representative: Biotronik Corporate Services SE

(57) **Abstract**

The present invention relates to a method for connecting a header assembly (1) to a housing (2) of an implantable medical device, the method comprising: providing a header assembly (1) having a first surface (1a) and providing a housing (2) of the implantable medical device, the housing (2) having a second surface (2a) to be connected to the first surface (1a), applying a film (3) that is thermally activatable to the first surface (1a) and/or to the second surface (2a), and pressing the first and the second surface (1a, 2a) with the film (3) therebetween against one another upon heating the film (3) to join the header assembly (1) to the housing (2).

## Description

The present invention relates to a method for connecting a header assembly to a housing of an implantable medical device and to an implantable medical device, particularly an implantable medical device that has been manufactured using the method according to the present invention.

In the prior art, bonding of header assemblies made of thermoplastic polyurethanes to the housing surface made of titanium with liquid silicone adhesives is known.

However, processing of liquid adhesives (Two-part liquid silicone adhesives) is usually difficult to automate due to the liquid state of the respective adhesive and the corresponding mixing and dosing process. Furthermore, the duration of the curing reaction is typically in the range of hours, the required temperatures are comparatively high. Further, the cured materials resulting therefrom usually have low mechanical strength.

Based on the above, the problem to be solved by the present invention is to provide a preferably media-tight, i.e., fluid-tight, connection between a header assembly and a housing of an implantable medical device, particularly between components made of titanium or a titanium alloy and polyurethane in the area of the header (e.g. interface between header and housing) of an implantable medical device, particularly of an active implant.

This problem is solved by a method having the features of claim 1 as well as by an implantable medical device having the features of claim 15. Preferred embodiments of these aspects of the present invention are stated in the corresponding dependent claims and are described below.

According to claim 1, a method for connecting a header assembly to a housing of an implantable medical device is disclosed, the method comprising:
- providing a header assembly having a first surface and providing a housing of the implantable medical device, the housing having a second surface to be connected to the first surface,
- applying an adhesive film that is thermally activatable to the first surface and/or to the second surface, and
- pressing the first and the second surface against one another with the respective film therebetween upon heating the respective film to join the header assembly to the housing.

Thus, the present invention allows to replace the liquid adhesive system by a solid adhesive system that may be applied automatically, wherein preferably the solid adhesive system comprises a thermally activatable (solid) adhesive film applied in advance to one or both joining partners. Heating of the entire system under pressure on the interface results in a structurally bonded material composite.

The term "thermally activatable film" is used within its meaning known to the skilled person; it particularly refers to an adhesive film that is little tacky or substantially tack-free until activated by heat. Furthermore, such thermally activatable films are also known as latent-reactive films, as for example disclosed in DE 10 2006 058527 A1. Non-limiting examples include thermally activatable adhesive polyurethane films,

Particularly, an adhesive single film may be applied to either the first surface or the second surface. Alternatively, an adhesive film may be applied to the first surface, and a further adhesive film may be applied to the second surface. As will be described further below, the respective adhesive film may be just placed adjacent the associated (first or second) surface or may be pre-fixed to the associated (first or second) surface.

According to an embodiment of the method according to the present invention, the adhesive film is a thermally activatable polyurethane film. Furthermore, preferably, the first surface of the header assembly is made out of a thermoplastic material, preferably thermoplastic polyurethane. Particularly, said thermoplastic polyurethane is one of:
Pellethane^{®} 2363-75D TPU, or Tecothane^{®} TT-1075D-M.

Particularly, in an embodiment of the method according to the present invention, the respective thermally activatable polyurethane film can be one of: DuploTEC^{®} 12233 SBF LTC or DuploTEC^{®} 12235 SBF LTC.

Furthermore, in a further embodiment of the method of the present invention, the respective thermally activatable adhesive film is a solid film that forms a thermoset material under heat exposure, particularly on a time scale of a few seconds.

According to yet another embodiment of the method according to the present invention, the second surface is formed out of a metal, particularly out of titanium or a titanium alloy. Particularly, in an embodiment, the titanium may be substantially pure titanium according to the standard ASTM B 265-00, i.e., the alloy comprises besides Ti, less than or equal to 0.20% Fe, less than or equal to 0.18% O, less than or equal to 0.08% C, less than or equal to 0.03% N, less than or equal to 0.015% H, wherein the rest is Ti. Particularly, the entire housing is formed out of the titanium, particularly according to said standard ASTM B 265-00.

According to an embodiment of the method according to the present invention, once the header assembly has been connected to the housing according to the method of the present invention, the header assembly may be electrically connected to a feedthrough of the housing. Thereafter, the header assembly may be provided with an outer surface layer by means of injection molding or by means of casting, e.g., with a cold-curing two-component epoxy resin. Alternatively, the header assembly may already comprise a finished outer surface layer and an electrical connection may be made to the feedthrough when the header assembly is connected to the housing (e.g., by using pluggable electrical contacts).

According to yet another embodiment of the method, the first and the second surface are pressed against one another with a pressure in the range from 15 N*cm⁻² to 75 N*cm⁻².

Further, according to an embodiment of the method, said pressing of the first and the second surface against one another is conducted at a temperature of the respective adhesive film in the range from 70° to 90°, particularly at a temperature of the respective adhesive film of about 80°, and/or wherein said pressing of the first and the second surface against one another is conducted over a time period of 50s to 70s, particularly over a time period of about 60s. Furthermore, according to a further embodiment of the method, providing the housing further comprises that the second surface of the housing is cleaned. In a further embodiment the second surface is pickled. In a further embodiment, the second surface is sandblasted. In yet a further embodiment, the second surface is wet-chemically oxidized. In another embodiment, the second surface is laser-structured. The afore-described processes can be combined as desired.

According to yet another embodiment of the method, providing the housing of the implantable medical device further comprises that the second surface of the housing is pretreated with an adhesion promoter. According to an embodiment of the method, the adhesion promoter is selected from the group comprised of: an alkoxysilane, a 3-aminopropyltrimethoxysilane (CAS No.: 13822-56-5). Particularly, the latter agent may be deposited from acetic acid ethanolic solution by dipping the second surface into the solution.

According to yet another embodiment of the method, providing the header assembly further comprises that the first surface of the header assembly is cleaned. In a further embodiment, the first surface is carbon dioxide snowblasted. In another embodiment, the first surface is plasma-activated (e.g. by means of an atmospheric pressure or low-pressure plasma).

In a further embodiment of the method according to the invention, applying the respective thermally activatable adhesive film comprises pressing the adhesive film on the first surface and/or the second surface, particularly with a pressure in the range from 15 N*cm⁻² to 75 N*cm⁻², and heating the respective thermally activatable adhesive film to provide initial adhesion of the respective adhesive film to the associated (first or second) surface. An adhesive single film may be pre-fixed in this manner to one of the surfaces. Alternatively, a film may be pre-fixed to each of the first and second surfaces, respectively. In a further embodiment, the respective adhesive film is heated to a temperature in the range from 40°C to 60°C, preferably to a temperature of about 50°C. According to yet another embodiment of the method, said heating is applied within a time period of 5s to 15s, preferably within a time period of about 10s.

According to a further embodiment of the method, applying the respective adhesive film to the associated (first or second) surface consists of arranging the respective adhesive film adjacent its associated (first or second surface) without applying heat to the film upon arranging it adjacent its associated surface prior to said pressing of the surfaces against one another.

According to yet another embodiment of the method, the implantable medical device is one of: an implantable (e.g. cardiac) pacemaker, an implantable cardioverter-defibrillator, an implantable neurostimulator; and/or wherein the header assembly is configured to provide an electrical connection between at least one electrode lead and the implantable medical device.

According to a further aspect of the present invention, an implantable medical device is disclosed, the implantable medical device comprising a housing and a header assembly, wherein the header assembly has been connected to the housing using the method according to the present invention.

According to yet another aspect of the present invention, an implantable medical device is disclosed, comprising a header assembly having a first surface and a housing having a second surface, wherein the first surface is preferably formed out of a thermoplastic polyurethane and wherein the second surface is preferably formed out of titanium or a titanium alloy (see e.g., above), wherein the first surface is connected to the second surface via at least one intermediate thermally activatable adhesive film, particularly a thermally activatable adhesive polyurethane film.

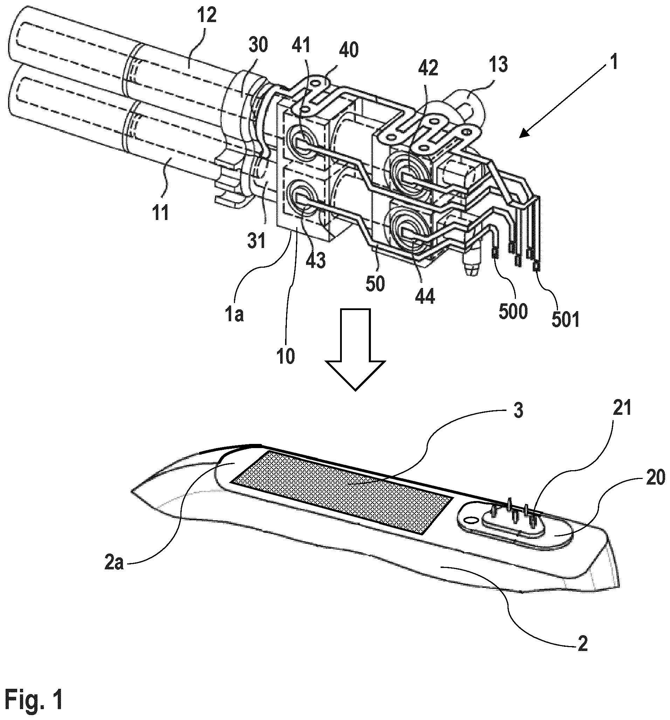

In the following, embodiments as well as further features and advantages of the present invention shall be described with reference to the Figure, wherein
- Fig. 1: shows an embodiment of a method according to the present invention.

Fig. 1 shows an embodiment of a method according to the present invention. The method allows to structurally bond a header assembly 1 of an implantable medical device to a housing 2 of the device, the housing 2 being preferably made of titanium or a titanium alloy or comprising at least a second surface 2a formed preferably out of titanium or a titanium alloy as described herein. The bonding process utilizes at least one thermally activatable adhesive film 3, preferably a thermally activatable polyurethane film. Particularly, a media-tight, i.e. fluid-tight, bond between the two joining partners, here a first surface 1a of the header assembly 1 on one side and the second surface 2a of the housing 2 on the other side may be achieved in the following manner.

The header assembly 1 may comprise a body 10 formed out of a thermoplastic polyurethane that forms a first surface 1a to be connected to the second surface 2a of the housing 2 via film 3. Furthermore, the header assembly 1 may comprise a first and a second receptacle 30, 31 for receiving a plug of an electrode lead, respectively. The header assembly 1 may comprise electrical contacts 41, 42, 43, 44 arranged on the body 10 for contacting plugs received in the respective receptacle. The contacts 41, 42, 43, 44 may be connected via metallic wire bands 50 and their contacts 500 to feedthrough-pins 21 of a feedthrough 20 of the housing 2 so as to connect the header contacts 41, 42, 43, 44 to an electronic circuit accommodated in the housing 2. Furthermore, the header assembly may comprise an antenna 40 arranged on the body 10, wherein the antenna 40 can be connected to a feedthrough-pin as well via a contact 501. The header assembly 1, particularly body 10, may be equipped with inserts or mandrels 11, 12, 13 that cover cavities such as the receptacles 30, 31 upon casting/injection molding.

Preferably, the second surface 2a of the housing 2 is made of titanium or a titanium alloy (e.g., ex. Grade 1, ASTM B 265-00, Ra 6.3 [arithmetic mean roughness]) and may be cleaned, particularly by automated cleaning with a mixture of water and a detergent, and subsequent rising with deionized water, pickled, particularly by treatment with a mixture with nitric acid and hydrofluoric acid to remove an oxide layer in the titanium substrate, pickled, particularly by treatment with a mixture with nitric acid and hydrofluoric acid to remove an oxide layer in the titanium substrate, sandblasted, wet-chemically oxidized, particularly oxidized with hydrogen peroxide and aqueous sodium hydroxide, and/or, in particular, laser-structured. Laser structuring may particularly performed with the following tools and parameters: Nb:YVO₄-laser or Nb:YAG laser with a wavelength of 1064 nm (e.g. Trumpf TM6130); Q-switch 20 kHz to 100 kHz; pulse duration with 60 kHz = approx. 20 ms; P_max = 20 W (direct beam, no power losses in beam splitter, camera flange, beam scanner; spot diameter at P = 100 % and 60 kHz = approx. 70µm. In addition, the second surface 2a is preferably pretreated with an adhesion promoter from the alkoxysilane class (e.g. 3-aminopropyltrimethoxysilane, CAS No.: 13822-56-5, deposited from acetic acid ethanolic solution by dipping).

The first surface 1a of the header assembly 1 to be bonded, which is made of thermoplastic polyurethane (e.g. Pellethane^{®} 2363-75D TPU or Tecothane^{®} TT-1075D-M ), may be cleaned, carbon dioxide snowblasted and/or, in particular, plasma-activated (atmospheric pressure or low-pressure plasma). Particularly, before the joining process, the thermally activatable polyurethane film 3 (e.g. DuploTEC^{®} 12233 SBF LTC or DuploTEC^{®} 12235 SBF LTC) is applied, particularly pressed, to one or both of the joining partners. As an example, shown in Fig. 1, the film 3 is placed on the second surface 2a of the housing 2 of the implantable medical device. This may be done under brief heating (e.g. 55 °C, 10s) and leads to initial adhesion of the film 3 to the second surface 2a. Alternatively, the assembly may be carried out without the influence of heat and pressure, in which case the film 3 only lies loosely on the second surface 2a. However, also the other film configurations described herein may be used (see above).

The joining process is carried out by pressing the two joining partners, i.e., the first surface 1a of the body 10 of the header assembly 1 and the second surface 2a together under brief heating (e.g. 15 - 75 N/cm² at 80°C for 60s) with the film 3 therebetween.

Particularly after the joining process, the contacts 500, 501 may be connected to the feedthrough-pins 21. An outer layer of the header assembly 1 may be provided by casting or injection molding material onto the header assembly 1 being connected to the housing via film 3.

The present invention offers the advantage that a processing of liquid adhesives can be avoided. Due to the solid connecting film, an easy, automated application of the solid adhesive film by "pick & place" is possible. The curing reaction may be initiated by a brief heating in the seconds range. A long annealing or "post-cure" process is no longer required. Furthermore, the formed connection has a higher mechanical strength than silicone.

## Claims

1. A method for connecting a header assembly (1) to a housing (2) of an implantable medical device, the method comprising:
- providing a header assembly (1) having a first surface (1a) and providing a housing (2) of the implantable medical device, the housing (2) having a second surface (2a) to be connected to the first surface (1a),
- applying an adhesive film (3) that is thermally activatable to the first surface (1a) and/or to the second surface (2a), and
- pressing the first and the second surface (1a, 2a) with the film (3) therebetween against one another upon heating the film (3) to join the header assembly (1) to the housing (2).

2. The method according to claim 1, wherein the film (3) is a thermally activatable polyurethane film, and wherein the first surface (1a) of the header assembly (1) is made out of a thermoplastic material, preferably thermoplastic polyurethane.

3. The method according to one of the preceding claims, wherein the second surface (2a) is formed out of titanium or a titanium alloy.

4. The method according to one of the preceding claims, wherein the first and the second surface (1a, 2a) are pressed against one another with a pressure in the range from 15 N/cm² to 75 N/cm².

5. The method according to one of the preceding claims, wherein said pressing of the first and the second surface (1a, 2a) against one another is conducted at a temperature in the range from 70° to 90°, particularly at a temperature of 80°, and/or wherein said pressing of the first and the second surface (1a, 2a) against one another is conducted over a time period of 50s to 70s, particularly over a time period of 60s.

6. The method according to one of the preceding claims, wherein providing the housing further comprises that the second surface (2a) is at least one of: cleaned, pickled, sandblasted, wet-chemically oxidized, laser-structured.

7. The method according to one of the preceding claims, wherein providing the housing (2) further comprises that the second surface (2a) is pretreated with an adhesion promoter.

8. The method according to claim 7, wherein the adhesion promoter is selected from the group comprised of: an alkoxysilane, a 3-aminopropyltrimethoxysilane.

9. The method according to one of the preceding claims, wherein providing the header assembly (1) further comprises that the first surface (1a) of the header assembly (1) is at least one of: cleaned, carbon dioxide snowblasted, plasma-activated.

10. The method according to one of the preceding claims, wherein said applying of the film (3) comprises pressing the adhesive film (3) on the first surface (1a) or the second surface (2a) and the heating the film (3).

11. The method according to claim 10, wherein said applying of the film (3) comprises that the film (3) is heated to a temperature in the range from 40°C to 60°C, preferably to a temperature of about 50°C, particularly within a time period of 5s to 15s, preferably within a time period of about 10s.

12. The method according to one of the claims 1 to 9, wherein said applying of the film (3) to the first and/or second surface (1a, 2a) consists of arranging the film (3) adjacent the first and/or second surface (1a, 2a) without heating the film (3) prior to said pressing of the first and the second surface (1a, 2a) against one another.

13. The method according to one of the preceding claims, wherein the implantable medical device is one of: an implantable pacemaker, an implantable cardioverter-defibrillator; an implantable neurostimulator and/or wherein the header assembly (1) is configured to provide an electrical connection between at least one electrode lead and the implantable medical device.

14. An implantable medical device comprising a housing (2) and a header assembly (1), wherein the header assembly (1) has been connected to the housing (2) using the method according to one of the preceding claims.

15. An implantable medical device comprising a housing (2) and a header assembly (1), the header assembly (1) having a first surface (1a) and the housing (2) having a second surface (2a), wherein the first surface (1a) is connected to the second surface (2a) via at least one intermediate thermally activatable adhesive film (3), particularly a thermally activatable polyurethane film.
